(19) Europäisches Patentamt
European Patent Office
Office européen des brevets

(11) Publication number: 0 358 940 B1

(12) EUROPEAN PATENT SPECIFICATION

(45) Date of publication of patent specification: 13.09.95

(51) Int. Cl.⁶: **C12N 15/60**, C07H 21/04, C12N 1/20, C12N 9/88, C12P 13/04, C12P 13/08, C12P 13/12, C12P 13/06, C12P 13/14, C12P 13/24, C12P 13/10, //(C12N1/20, C12R1:15),(C12N1/20, C12R1:13)

(21) Application number: 89114632.6

(22) Date of filing: 08.08.89

(54) DNA fragment coding for phosphoenolpyruvat corboxylase, recombinant DNA carrying said fragment, strains carrying the recombinant DNA and method for producing L-aminino acids using said strains.

(30) Priority: 12.09.88 GB 8821319

(43) Date of publication of application:
21.03.90 Bulletin 90/12

(45) Publication of the grant of the patent:
13.09.95 Bulletin 95/37

(84) Designated Contracting States:
BE DE ES FR GB IT NL SE

(56) References cited:
EP-A- 0 143 195
EP-A- 0 179 338
FR-A- 2 581 653

(73) Proprietor: Degussa Aktiengesellschaft
Weissfrauenstrasse 9
D-60311 Frankfurt (DE)

(72) Inventor: Bachmann, Bernd, Dr.
Meyerfeld 10a
D-4806 Werther (DE)
Inventor: Thierbach, Georg, Dr.
Gunststrasse 21
D-4800 Bielefeld 1 (DE)
Inventor: Kalinowski, Jörn, Dr.
Drögestrasse 25
D-4800 Bielefeld (DE)
Inventor: Pühler, Alfred, Prof. Dr.
Am Waldschlösschen 2
D-4800 Bielefeld 15 (DE)
Inventor: O'Reagan, Mike, Dr.
FIDIA, Via Ponte della Fabbrica 3/A
I-35031 Abano Terme, PADOVA (IT)
Inventor: Viret, Jean-Francois
Gartenstrasse 50
CH-3177 Laupen (CH)

Inventor: **Lepage, Pierre**
**1 rue de la Division Leclerc**
**F-67000 Strasbourg (FR)**
Inventor: **Lemoine, Yves**
**4 rue des Alisiers**
**F-67000 Strasbourg-Neudorf (FR)**

## Description

The enzyme phosphoenolpyruvate carboxylase (EC 4.1.1.3.1 ; PEPC) is of particular interest in the metabolism of amino acids, since it is involved in a so-called anaplerotic function, which ensures a constant supply of oxaloacetate to the cell.

Oxaloacetate in turn occupies a central position in the metabolism of amino acids, both as the immediate precursor of L-aspartate and as a member of the tricarboxylic acid (TCA) cycle. Indeed, amino acids such as L-lysine, L-methionine, L-threonine and L-isoleucine derive from L-aspartate in a series of branched and highly interregulated biosynthetic pathways, whereas amino acids such as L-glutamate, L-glutamine, L-proline, L-arginine, L-citrulline, L-ornithine etc. derive from intermediates of the TCA cycle.

Thus, PEPC ativity is implicated in the biosynthesis of all the amino acids hereabove mentionned.

It is apparent from the above considerations that the biosynthetic levels of amino acids, such as L-lysine, might vary depending on the intra-cytoplasmic specific activities of enzymes such as PEPC.

Considering the important role played by PEPC in the biosynthesis of amino acids, it has always been desirable to try to provide improved means for the production of amino acids by increasing the activity of PEPC.

For example, European Patent Application EP-A-0143195 discloses the cloning of the ppc gene isolated from Brevibacterium lactofermentum ATCC 13869 and the transformation of bacteria of the genera Corynebacterium with a recombinant plasmid, carrying said gene in order to produce L-lysine or threonine.

The prior art has also described Corynebacterium melassecola strains transformed with a recombinant plasmid DNA carrying the ppc gene of C.melassecola.

These strains show an increased PEPC activity, but no evidence is provided for an increase of amino acid production (FR-A-2581653).

There is no suggestion in these publications to enhance the fermantative production of amino acids, especially L-lysine, by cloning the ppc gene isolated from Corynebacterium glutamicum.

Summary of the Invention:

The inventors have discovered that, when introducing the genetic information coding for PEPC into an appropriate vehicle capable of replicating in Corynebacteria or Brevibacteria, and the resulting hybrid vehicle carrying said genetic information is replicated in an appropriate Corynebacterium or Brevibacterium host or recipient, the transformed microorganism is an excellent producer of L-lysine, L-threonine and L-isoleucine, especially L-lysine.

This invention is of particular interest since many strains of Brevibacterium and Corynebacterium genera producing high amounts of the above mentioned L-amino acids can be utilized as hosts.

The invention thus relates to a process for the fermentative production of L-amino acids and to the various genetic and microbiological elements involved in said process. For example, the invention relates to the isolated form of the gene for PEPC, to various vehicles containing said gene, which vehicles are replicable in above mentioned bacteria, to various microbes of said microorganisms containing such vehicles, and to various fermentation processes for the production of L-amino acids.

Description of the Preferred Embodiments:

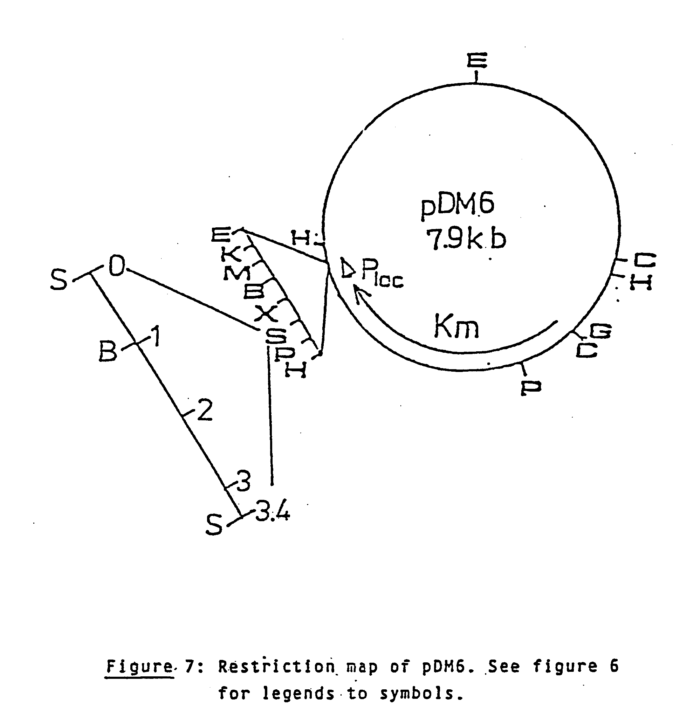

The present invention relates to Plasmid pDM6, introduced in C.glutamicum DM58-1 which is deposited at the DSM under DSM 4697 characterised by containing a genetic sequence comprising information coding for the production of a protein having the activity of phosphoenol-pyruvate carboxylase (PEPC), having a length of 7,9 kb and a restriction map as shown in Fig. 7.

The DNA fragment consists essentially of 3422 base pairs, is flanked at its terminii by Sal I restriction sites and is coding for the production of PEPC, which shows the N-terminal amino acid sequence Thr[1] - Asp - Phe - Leu - Arg[5] - Asp - Asp - Ile - - Arg - Phe[10] -Leu - Gly - Gln - Ile - Leu[15].

The structural gene coding for PEPC consists of 2757 base pairs.

The PEPC of interest is not stimulated by acetyl CoA.

A recombinant DNA containing a DNA fragment consisting of the ppc gene can be constructed according to conventional methods for example, by digesting chromosomal DNA and vector plasmid with a restriction enzyme, followed by treatment with a DNA ligase or by digesting chromosomal DNA and vector plasmid with a restriction enzyme, followed by treatment of cleaved terminals with terminal transferase, DNA polymerase, etc., and succesive treatment with a DNA ligase, etc. (Methods in Enzymology 68 (1979)-).

To isolate the ppc gene, a genomic bank of C.glutamicum ATCC 13032 was constructed in the plasmid pUC18, a vector commonly used for cloning in the gram negative bacterium E. coli. The ppc gene was isolated by complementation of known E. coli mutant strains affected in the corresponding genes. Candidates clones were analysed and shown both genetically and enzymatically to bear inserts containing the sought after gene.

The ppc gene was later subcloned onto so-called plasmid shuttle vectors (vehicles) allowing its propagation in both E., coli and the original Corynebacterium host, or any type of glutamate producing strain, in order to provide a recombinant DNA molecule containing the new DNA fragment inserted in a vector capable of replication in a glutamate producing strain.

Of particular interest are the vectors pZ1 and pCV 22.

Plasmid pZ1 comprises a drive unit region capable of propagating in Coryneform glutamic acid producing bacteria and E.coli, and having at least a region to express resistance to a drug.

pZ1 is disclosed in the German Patent Application 37 37 729.9.

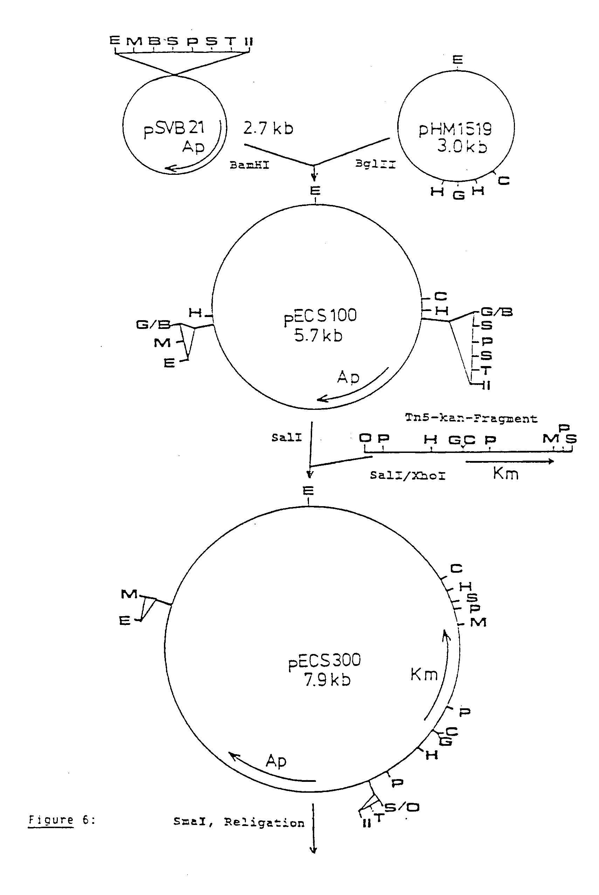

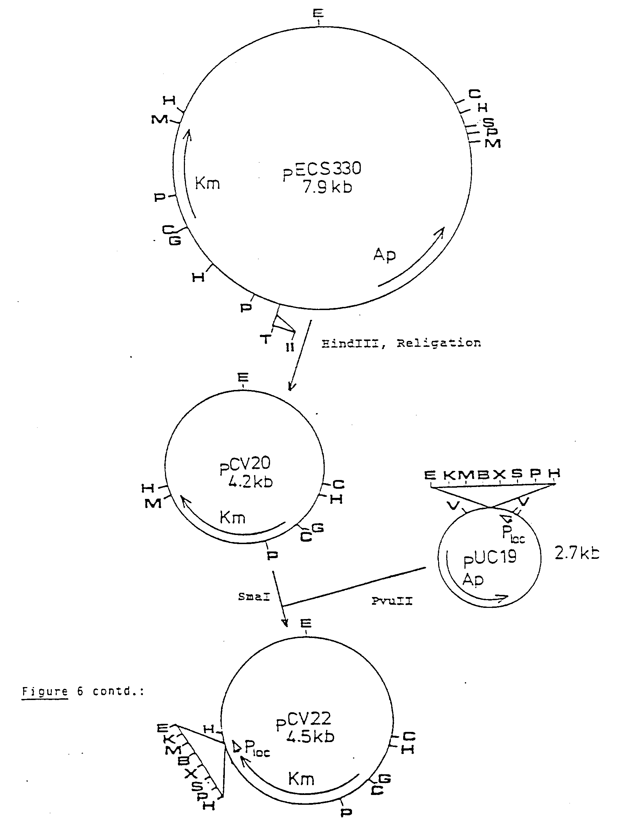

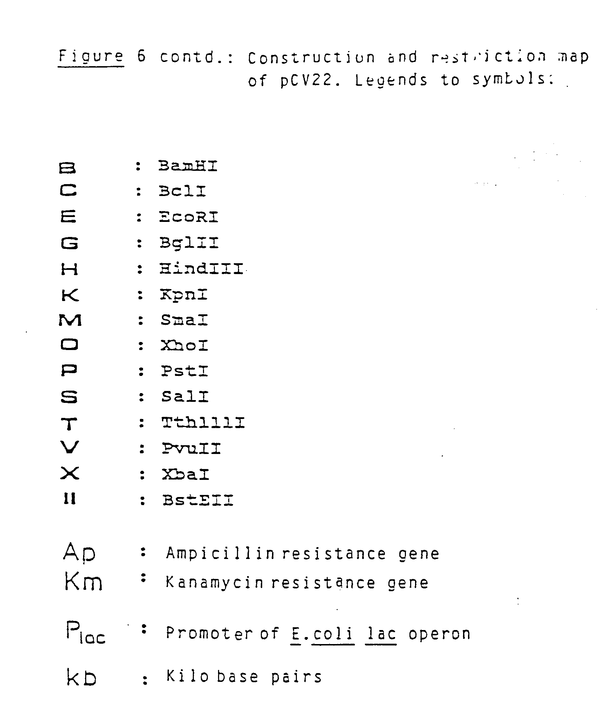

pCV 22 is an essentially pure plasmid, which is characterized by a length of 4,5 kb and a restriction endonuclease clevage chart shown in Figure 6.

The vectors can be obtained from the cells of microorganisms on deposit, by lysing the cells according to the state of art.

The recombinant DNA containing the ppc gene of wild type or mutant type can be introduced into microorganisms preferred of the genus Corynebacterium or Brevibacterium by known transformation methods.

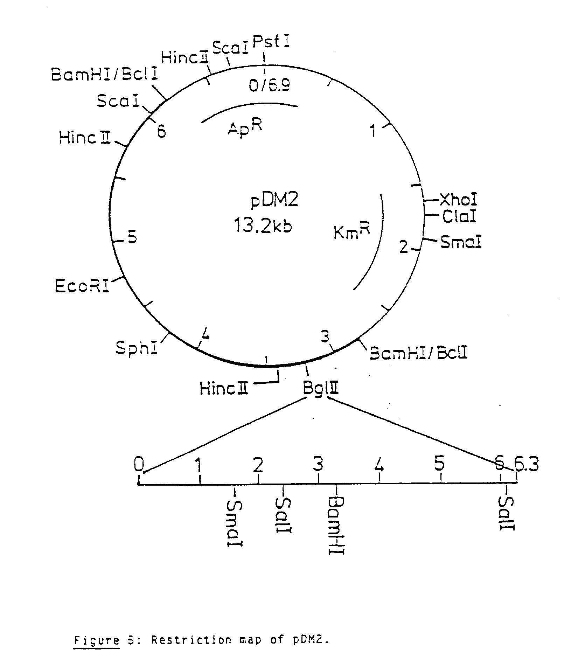

Vehicles capable of replication in said microorganisms are the plasmids pDM 2 or pDM 6 (restriction maps shown in Figure 5 and 7).

C.glutamicum, C.melassecola, B.lactofermentum and B.flavum are preferred as recipients or hosts, especially bacteria already known for the production of amino acids.

For the expression of the ppc gene in the transformed strains, any promoter known as efficient in Corynebacterium or Brevibacterium can be used. They may be endogenous promoters of these strains, that is promoters controlling the expression of genes originally belonging to the strain. They may also be exogenous promoters, among which the promoter ptac, plac, ptrp, $P_R$ and $P_L$ of phage $\lambda$, can be mentionned.

A further object of the invention is a process for the production of an amino acid selected especially from the group L-lysine, L-threonine, L-isoleucine, especially L-lysine.

The methods of culturing the L-amino acid producing strains thus obtained are conventional, and are similar to the methods for the cultivation of known L-amino acids producing microorganisms.

The culture medium employed can be a conventional medium containing carbon sources, nitrogen sources, and organic ions and, when required, minor organic nutrients such as vitamins and amino acids. Examples of suitable carbon sources include glucose, sucrose, lactose, starch hydrolysate and molasses. Gaseous ammonia, aqueous ammonia, ammonia salts and other nitrogen containing materials can be used as the nitrogen source.

Cultivation of the transformed organisms containing the vehicle carrying the ppc gene is conducted under aerobic conditions in which the pH and the temperature of the medium are adjusted to a suitable level and continued until the formation of L-amino acid ceases. In a preferred embodiment a transformed Corynebacterium glutamicum is used, selected from the group of those having the identifying characteristics of Corynebacterium glutamicum DSM 4697 deposited under the provisions of the Budapest Treaty on July 8, 1988 at Deutsche Sammlung von Mikroorganismen (DSM).

Another strain of interest is a Brevibacterium, selected from the group of those having the identifying characteristics of B.flavum DSM 5399.

The amino acids, which accumulate in the culture medium, can be recovered by conventional procedures.

By the methods of the present invention, above mentioned L-amino acids, especially L-lysine, can be produced in higher yields than has been achieved in previously known methods using artificial mutants of Brevibacterium and Corynebacterium.

Having generally described this invention, a further understanding can be obtained by reference to certain specific examples, which are provided herein for purposes of illustration only, and are not intended to be limiting, unless otherwise specified.

**1. Isolation of the <u>Corynebacterium glutamicum</u> ATCC13032 <u>ppc</u> gene.**

**1.1 Construction of genomic bank of <u>C. glutamicum</u> ATCC13032**

Total DNA from <u>C. glutamicum</u> ATCC13032 was isolated as described by Chater <u>et al</u>. (Curr. Topics Microb. Immunol. <u>96</u>, 69 pp (1982)) and partially digested with <u>Sau3</u>AI. Fragments ranging in size from 4-20 kb were purified from low melting temperature agarose. The DNA solution, was dialyzed against 2 L of TE buffer (Tris 10 mM, EDTA 1 mM). 2 $\mu$g of size fractionated chromosomal DNA was ligated using T4 DNA Ligase with 1 $\mu$g of plasmid pUC18 (Yanish-Perron, C. <u>et al</u>. (1985) Gene <u>33</u>, 103 pp) which had been digested with <u>Bam</u>HI and treated with alkaline phosphatase.

<u>E. coli</u> NM522 (Gough, J.A. and Murray, N.E. (1983) J. Mol. Biol. <u>166</u>, 1 pp) was transformed according to Hanahan (J. Mol. Biol. <u>166</u>, 557 pp (1983)) with the ligation mixture and transformants were selected at 37ºC on LB agar plates (Davis, R.W. et al. (1980) Advanced Bacterial Genetics, Cold Spring Harbor Laboratory) containing ampicillin (100 $\mu$g/ml) and 5-bromo-4-chloro-indolyl-$\beta$-D-galactopyranoside (X-gal, 50 $\mu$g/ml). 80 % of clones were colourless on X-gal plates, indicating the presence of inserted DNA. To estimate more precisely the efficiency of cloning, plasmid DNA from 46 colourless clones was examined by restriction enzyme digestion : 7 % of clones contained no inserts; 41 % of clones contained small inserts with an average size of 0.5 kb; 52 % of clones contained inserts ranging in size from 1.35 kb to 8.5 kb with an average size of 5 kb.

In total $10^4$ clones were obtained upon transformation of strain NM522. These clones were pooled in 4 families (CgSA, CgSB, CgSC and CgSD) and plasmid DNA was prepared by CsCl-EtBr density gradient centrifugation.

**1.2 Cloning of the <u>ppc</u> gene**

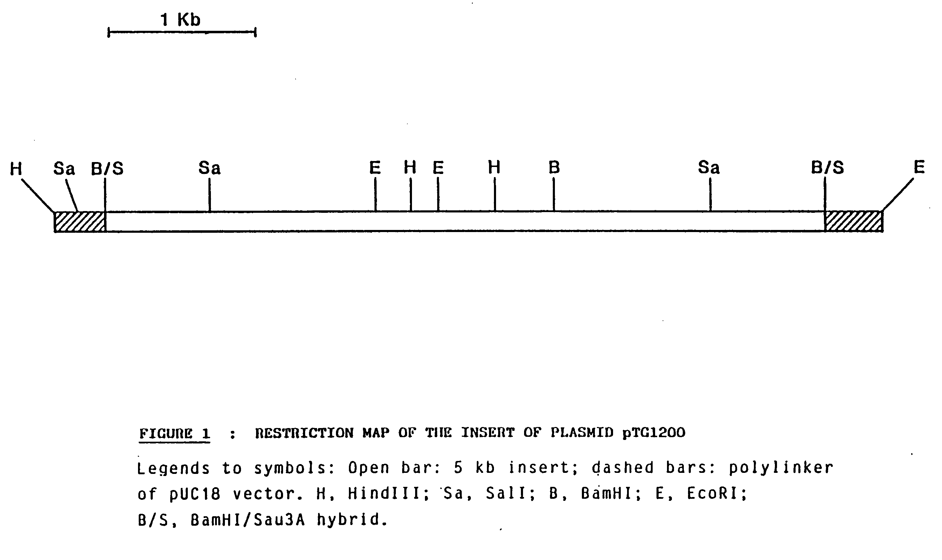

Competent cells of <u>E. coli</u> XH11 (Mountain, A. <u>et al</u>. (1984) Mol. Gen. Genet <u>197</u>, 82 pp) were transformed with 5 x 200 ng of each of the families of the <u>C. glutamicum</u> gene bank. Transformation mixes were spread on M9 agar (Miller, J.H. (1972) Experiments in Molecular Genetics, Cold Spring Harbor Laboratory) plus arginine (50 $\mu$g/ml) and ampicillin (100 $\mu$g/ml) and on LB plus ampicillin (100 $\mu$g/ml). A transformation frequency of $10^4/\mu$g was obtained on LB plus ampicillin. 108 clones were isolated on M9 agar containing arginine and ampicillin from the transformation with family CgSD. Restriction enzyme digestion of isolated DNA indicated that all clones contained the same plasmid. The plasmid (pTG1200) consisted of pUC18 plus an insert of approximately 5 kb. A restriction map of the insert of pTG1200 is shown in Figure 1. Retransformation of XH11 by pTG1200 led to complementation of the <u>ppc</u> mutation. Southern hybridisation (Maniatis, T. <u>et al</u>. (1982) Molecular Cloning, Cold Spring Harbor Laboratory) confirmed that chromosomal DNA of <u>C. glutamicum</u> ATCC13032 had been cloned in pTG1200.

**1.3 Localization of the <u>ppc</u> gene on the cloned DNA fragment.**

To better localize the <u>ppc</u> gene, subcloning experiments were undertaken. Digestion of pTG1200 with <u>Sal</u>I generates an internal fragment of 3.5 kb (Figure 1). pTG1200 digested with <u>Sal</u>I was ligated with <u>Sal</u>I cut pBR322 (Bolivar, F. <u>et al</u>. (1974) Gene <u>2</u>, 95 pp). To eliminate religated pTG1200 molecules the ligation mixture was digested with <u>Xba</u>I. <u>E. coli</u> XH11 was transformed with the ligation mixture and plated on M9 agar containing arginine and ampicillin. Plasmid DNA of complemented clones was examined and found to consist of pBR322 plus the 3.5 kb <u>Sal</u>I fragment of pTG1200.

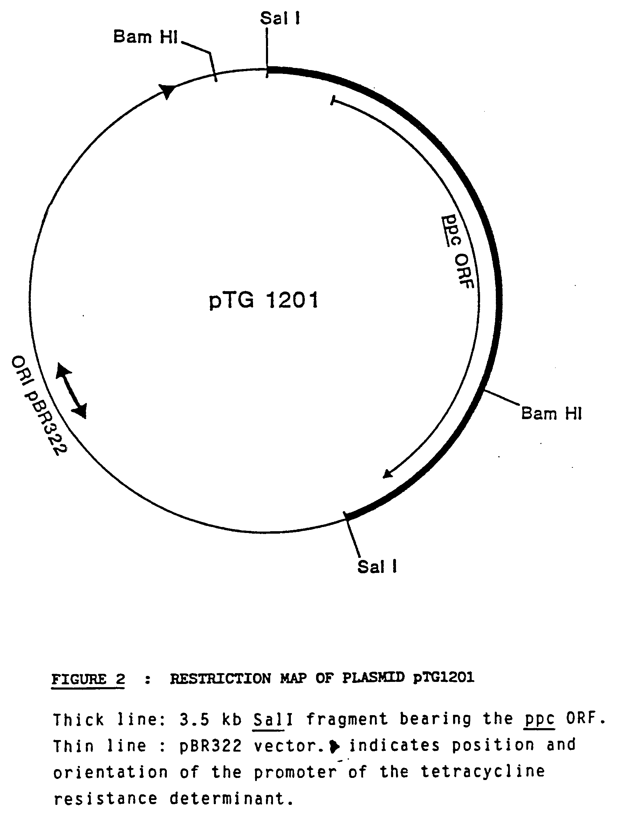

The constructed plasmid was designated pTG1201 (Figure 2) and the corresponding strain XH11/pTG1201.

**1.4 Measurement of enzyme activity in <u>C. glutamicum</u> ATCC13032 and in <u>E. coli</u> clones carrying a recombinant plasmid containing the DNA fragment with the <u>C. glutamicum</u> <u>ppc</u> gene.**

Phosphoenolpyruvate carboxylase activity was assayed in <u>C. glutamicum</u> ATCC13032 and in <u>E. coli</u> strain XH11/pTG1201. <u>E. coli</u> strain MM294 (Hanahan, D. (1983) J. Mol. Biol. 166, 557 pp) was used as a positive control and strain XH11 as negative control. <u>C. glutamicum</u> ATCC13032 was cultivated in MMYE medium (Katsumata, R. <u>et al</u>. (1984) J. Bact. <u>159</u>, 306 pp) and <u>E. coli</u> strains in M9 medium supplemented with arginine (50 $\mu$g/ml) and ampicillin (100 $\mu$g/ml) in the case of XH11/pTG1201, with thiamine (200 $\mu$g/l) in the case of MM294 and with sodium succinate (5 g/l) and arginine (50 $\mu$g/ml) in the case of XH11. The growth conditions were 37ºC and 150 rpm for <u>E. coli</u> strains and 30ºC and 150 rpm for <u>C. glutamicum</u>.

The cultures were harvested by centrifugation at the beginning of the stationary phase of growth and washed three times with a buffer composed of 100 mM Tris/Hcl pH 7.5 and 1 mM DTT. Disruption of the cells was performed in a glass bead mill (MSK-Homogenisator; B. Braun Melsungen, FRG). PEP carboxylase activity in the clear supernatents was determined after extensive dialysis against 100 mM Tris/Hcl pH 7.5; 0.8 M (NH$_4$/SO$_4$; 1 mM DTT. The modified malate dehydrogenase coupled assay (Ozaki, H. and Shiio, J. (1969) J. Biochemistry 66, 297 pp) was used and NADH disappearance was followed photometrically at 340 nm. The assay mixture included the following components: 6 mM PEP; 10 mM NaHCO$_3$; 100 mM Tris/HCl pH 7.5; 0.15 mM NADH; 2 U/ml malate dehydrogenase (pig heart); 3.3 mM MnSO$_4$ and PEP carboxylase preparation in a final volume of 1 ml . Unspecific NADH decomposition was measured before starting the reaction by addition of Mn$^{++}$. Protein concentration was determined by the methods of Lowry et al. (Lowry.O.H. et al. (1951) J. Biol. Chem. 193, 265 pp) or Bradford et al. (Bradford, M.M. (1976) Analyt. Biochem. 72, 248 pp).

The data shown in Table 1 confirm that the 3.5 kb Sall DNA fragment cloned from C. glutamicum ATCC13032, contained in plasmid pTG1201, encodes the phosphoenolpyruvate carboxylase gene.

In particular, the effect of acetyl-CoA known to stimulate phosphoenolpyruvate carboxylase from E. coli (Izui. K. et al. (1981) J.Biochem. 90, 1321 pp) and Brevibacterium flavum (Ozaki, H. et al. (1969) J. Biochem. 66, 297 pp) was investigated. From the results shown in Table 1 it is evident that phosphoenolpyruvate carboxylase from C. glutamicum ATCC13022, either produced in its original host or as a result of cloning into E. coli to form strain XH11/pTG1201, is not stimulated by acetyl-CoA under the assay conditions described above.

| Strains | Phosphoenolpyruvate carboxylase activity (U/mg protein) | |
| --- | --- | --- |
| | without acetyl-CoA | in the presence 0.2 mM acetyl-CoA |
| C. glutamicum ATCC13032 | 0.226 | 0.225 |
| E.coli MM294 | 0.035 | 0.158 |
| E. coli XH11/pTG1201 | 1.010 | 1.090 |
| E.coli XH11 | 0.0 | 0.0 |

Table 1: Phosphoenolpyruvate carboxylase activity in dialyzed homogenates obtained from C. glutamicum and from different E. coli strains.

### 2. Determination of the nucleotide sequence of the ppc gene of C. glutamicum ATCC13032.

The nucleotide sequence of the entire 5 kb insert of pTG1200 was sequenced using a shot gun approach (Messing,J. et al. (1981) Nucleic Acids Res. 9, 309 pp). The Smal site of pTG1200 was replaced by an Xbal site by cloning of a blunt ended oligomer (GTGTCTACAGTG) to generate a new plasmid called pTG1202. 10 µg of the 5.0 kb Xbal insert of pTG1202 was purified from low-melting-temperature agarose. The fragment was re-circularised using T4 DNA ligase, randomly fragmented by sonication and finally blunt ended using Klenow polymerase in the presence of 2 mM of each dATP, dGTP, dCTP and dTTP. Fragments ranging from 300-800 bp in size were isolated from low melting point agarose and ligated with Smal digested M13mp8 phage (Messing, J. and Vieira, J. (1982), Gene 19, 269-276). 160 clones were sequenced by the chain termination method (Sanger, F. et al. (1977) Proc. Natl. Acad. Sci. USA 74, 5463 pp) and overlapping clones indentified by computer-aided analysis (DNASTAR, Inc., 1801 University Ave, Madison, WI53705, USA). The entire sequence of the inserted DNA fragment contained in pTG1202 bearing the ppc gene of C. glutamicum ATCC13032 is shown in Figure 3.

6

The entire sequence which has been determined is 4885 bp in length. A search for possible protein encoding regions has revealed a long open reading frame (ORF) showing homology to ppc gene sequences from E. coli (Fujita, N. et al. (1984) J. Biochem. 95, 909 pp) and other organisms (Katagiri, F. et al. (1985) Gene 38, 265 pp and Izui, K. et al. (1986) Nucleic Acids Res. 14, 1615 pp) indicating that this ORF encodes the PEP carboxylase gene. This ORF is contained within the 3.5 kb SalI DNA fragment extending between coordinates 652 and 4077, cloned in pTG1201. Sequence analysis identified two possible start sites of translation (coordinates 921 and 906) giving rise to a protein product of either 919 or 924 amino acids respectively.

**2.1 Determination of the N-terminal sequence of the PEP carboxylase protein.**

In order to precisely identify the initiation codon of the ppc gene product, the N-terminal amino acid sequence of the PEP carboxylase protein was determined.

Purification of PEP carboxylase from C. glutamicum ATCC13032 was performed after disruption of the cells using a glass bead MSK-Homogenisator. After precipitation of nucleic acids by 0-3% Streptomycine sulphate, a fractionated $(NH_4)_2 SO_4$ (0-50% and 50-70%) precipitation was performed. The redissolved pellet from the 50-70% $(NH_4)_2 SO_4$ precipitation was dialyzed against starting buffer (20 mM Tris/Hcl pH 7.5; 100 mM $(NH_4)_2 SO_4$; 1 mM DTT) and further purified by ion exchange chromatography on Q Sepharose fast flow. Gel-filtration on Sephacryl S300 superfine was then performed with the concentrated PEP carboxylase fractions: elution was with stabilizing buffer (100 mM Tris/Hcl pH 7.5; 800 mM $(NH_4)_2 SO_4$; 1 mM DTT). Finally, samples were processed through an affinity chromotography step on Blue Sepharose, eluted using L-aspartate (70 mM) and dialyzed against stabilizing buffer to remove aspartate. The homogenous PEP carboxylase fractions - as proved by SDS-PAGE - were then concentrated by a factor of about four using a speed-vac concentrator at 5°C.

Desalting of enzyme fractions (1 ml containing about 25 $\mu$g of protein) was performed by dilution with 5 ml TRIS/HCl 100 mM pH 7.5. Samples were then concentrated to a final volume of 1.5 with a 8400 Amicon concentrator using a YM30 membrane (2.5 cm diameter). This procedure was repeated twice. The concentrated solution was divided into three (2 ml) Eppendorf tubes. Protein precipitation was then performed at -80°C 48 h after addition of ethanol (2 ml) in each Eppendorf tube. Pellets were pooled, washed twice with -80°C cooled ethanol/water (80/20 V/V). Samples were loaded on a precycle filter for sequencing after solubilization in formic acid. Sequencing was performed on an Applied Biosystem 470A protein sequencer with an on-line 120A PTH-analyser.

The obtained sequence is the following:

Thr[1]-Asp-Phe-Leu-Arg[5]-Asp-Asp-Ile-Arg-Phe-[10]-Leu-Gly-Gln-Ile - Leu[15]

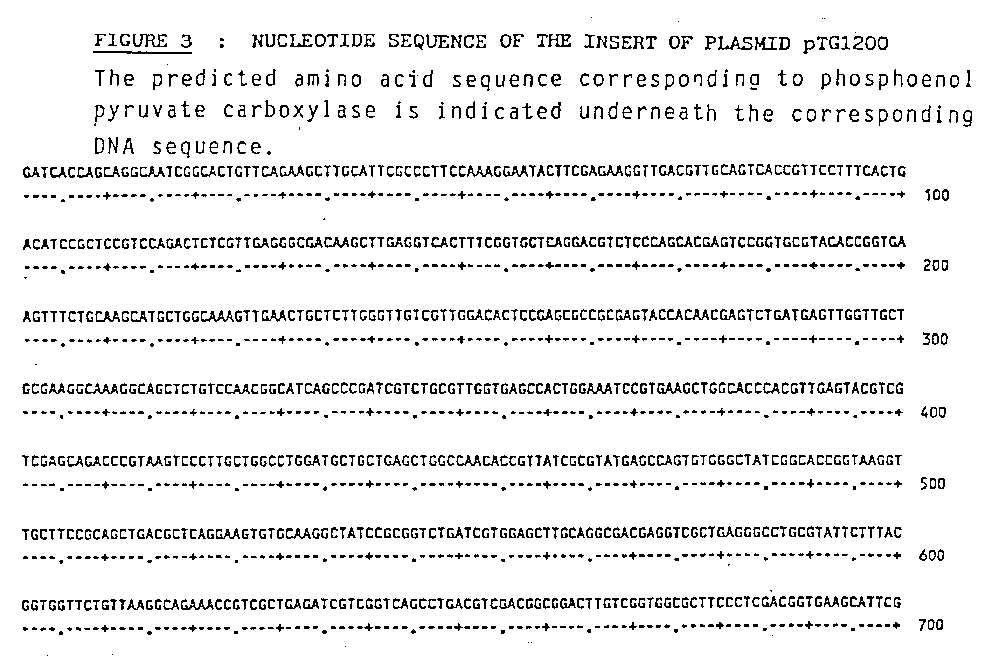

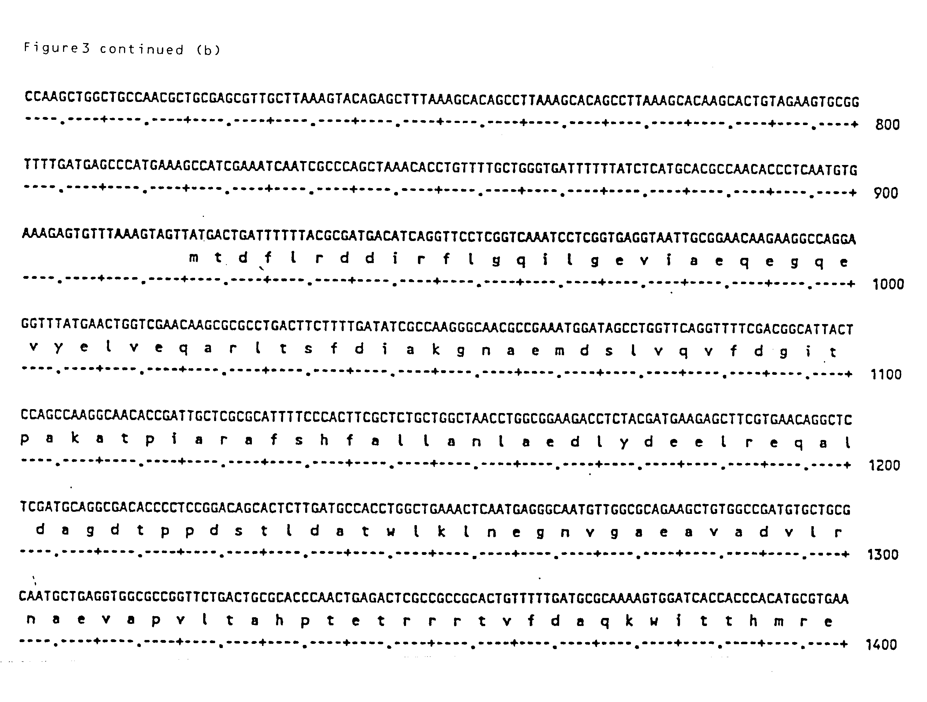

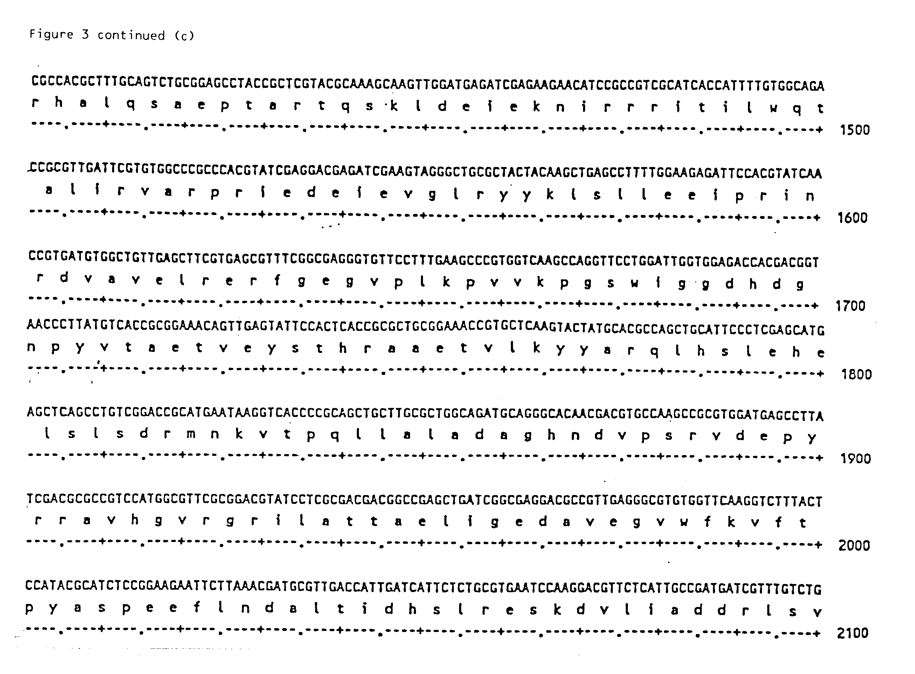

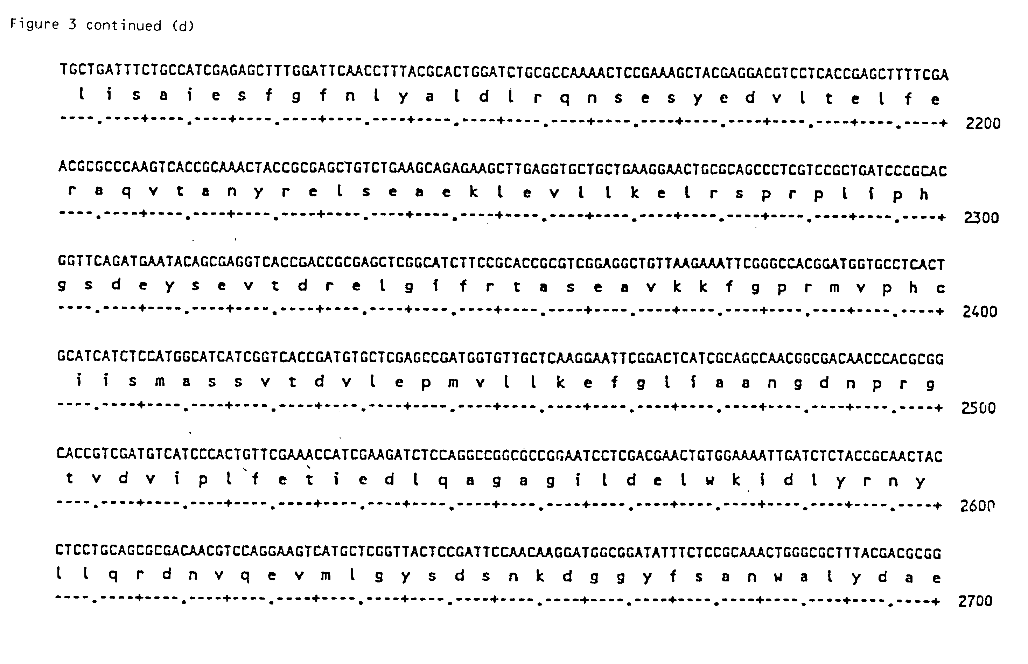

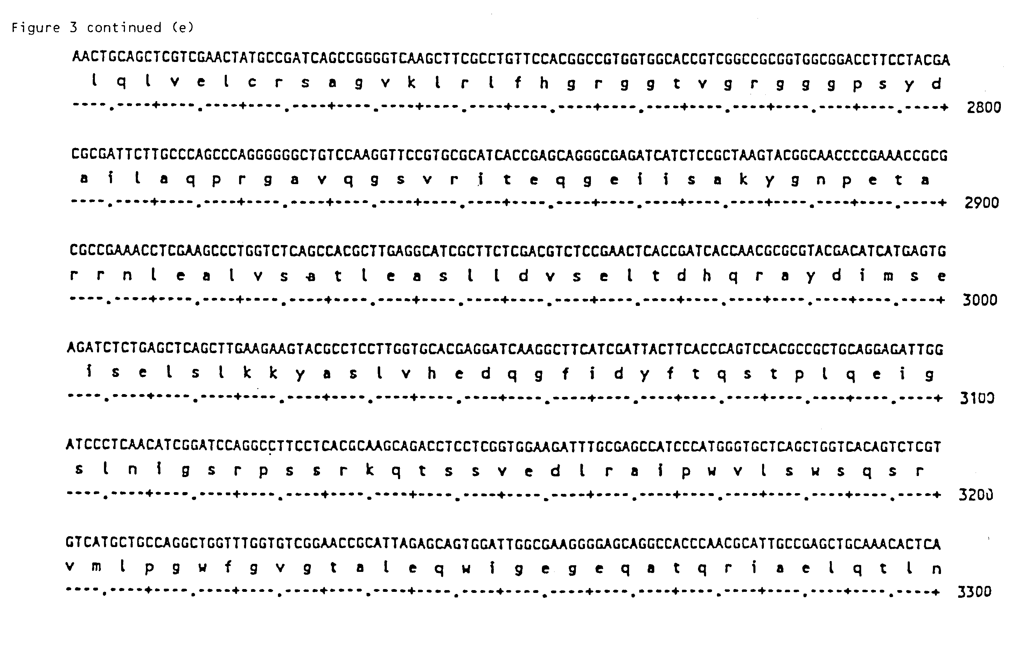

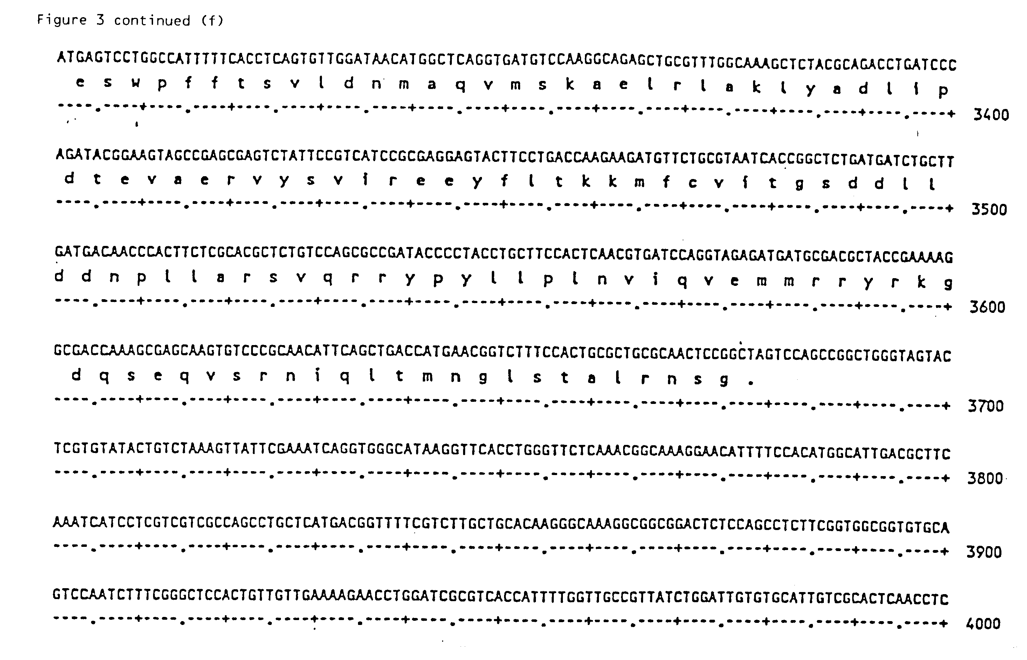

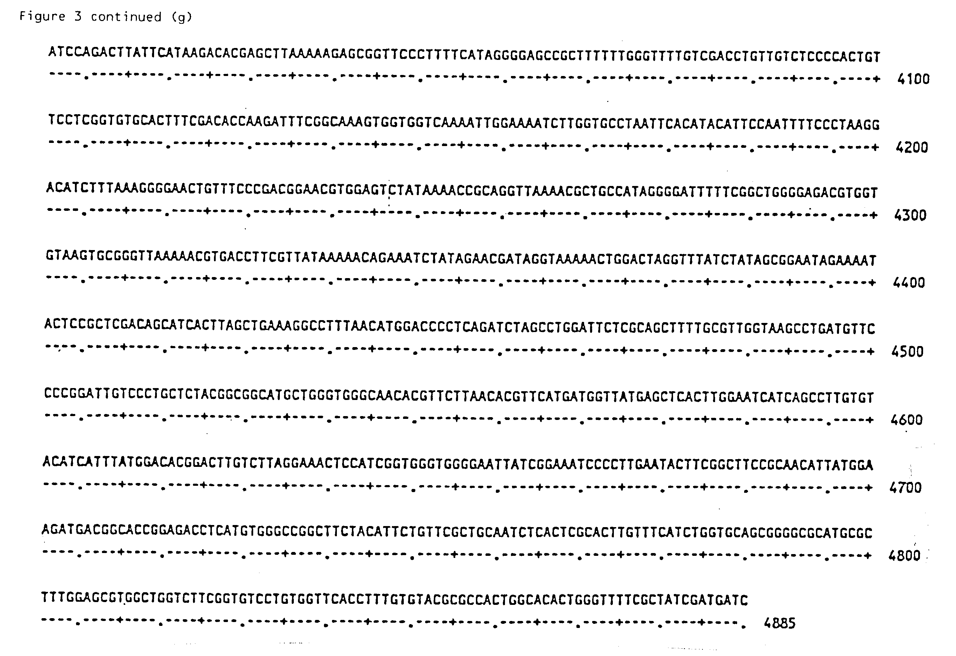

This result shows that PEP carboxylase is encoded by the ORF of 919 amino acids stretching between the ATG codon at position 921 and the TAG codon at position 3678. The initiation codon lies about 14 base pairs downstream from a putative Shine-Dalgarno sequence (coordinates 900-908, Figure 3).

**3. Cloning and expression of the ppc gene from C. glutamicum ATCC13032 in C. glutamicum ATCC13032.**

**3.1 Construction of the C. glutamicum/E. coli shuttle vector pZ1.**

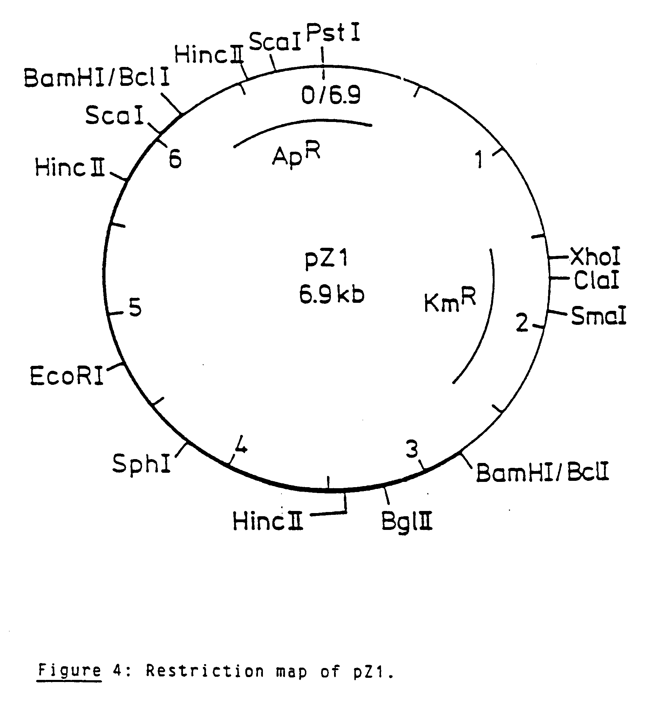

The structure of the plasmid shuttle vector pZ1 is shown in figure 4. It was constructed from E. coli vector pACYC177 (Chang, A.C.Y. and Cohen, S.N. (1978) J. Bact. 134, 1141 pp) and from C. glutamicum plasmid pHM1519 (Miwa, K. et al. (1984) Agric. Biol. Chem. 48, 2901 pp) as disclosed in Deutsche Patentanmeldung 3737729.9 E. coli strain DM272-3 containing plasmid vector pZ1 was deposited at the Deutsche Sammlung von Mikroorganismen under DSM4242.

**3.2 Cloning of the ppc gene onto the C. glutamicum/E. coli shuttle vector pZ1.**

Plasmid pTG1200 was isolated from E. coli XH11/pTG1200 and partially digested with Sau3A and plasmid pZ1 isolated from DM272-3 (= DSM4242) was linearized with BgIII. Both DNAs were mixed, ligated with T4 DNA Ligase and the ligation mixture used to transform E. coli XH11. Transformants were selected on M9 agar containing arginine (50 $\mu$g/ml) and Kanamycin (10 $\mu$g/ml). Plasmid-DNA from one of the transformants called XH11/pDM2 was isolated and characterized by restriction mapping. The structure of pDM2 is shown in Figure 5. Enzyme measurements revealed that strain XH11/pDM2 had a phosphoenol-pyruvate carboxylase activity which was not stimulated by acetyl-CoA (Table 2) as was shown in Table 1 for

strain XH11/pTG1201.

| Strains | Phosphoenolpyruvate carboxylase activity (U/mg protein) | |
| --- | --- | --- |
| | without acetyl-CoA | in the presence of 0.2 mM acetyl-CoA |
| C. glutamicum ATCC13032 | 0.226 | 0.225 |
| E. coli XH11 | 0.0 | 0.0 |
| E. coli XH11/pDM2 | 0.261 | 0.272 |

**Table 2:** Phosphoenolpyruvate carboxylase activity in dialyzed homogenates obtained from C. glutamicum ATCC13032 and from E. coli strains XH11 and XH11/ pDM2.

### 3.3 Construction of the C. glutamicum vector pCV22.

Plasmid pHM1519 (Miwa, K. et al. (1984) Agric. Biol. Chem. 48, 2901 pp) isolated from C. glutamicum ATCC13058 was cut with BglII and ligated to the pUC8 (Vieira, J. and Messing, J. (1982) Gene 19, 259 pp) derivative pSVB21 (Arnold, W. et al. Gene (1988) 70, 171 pp) cut with BamHI. The ligation mixture was used to transform E. coli strain JM83 (Vieira, J. and Messing, J. (1982) Gene 19, 259 pp) and transformants were selected on LB agar containing ampicillin. Plasmid DNA was isolated from one of the transformants and called pECS100.

The Kanamycin resistance gene of Tn5 (Jorgensen, R.A. et al. (1979) Mol. Gen. Gen. 177, 65 pp) was isolated as an XhoI-SalI DNA fragment from a pACYC184 (Chang, A.C.Y. and Cohen, S.N. (1978) J. Bact. 134, 1141 pp) derivative carrying Tn5 and inserted into the SalI site of pECS100 to form pECS300 which was transferred to C. glutamicum ATCC13032 by transformation (Yoshihama, M. et al. (1985) J. Bact. 162, 591 pp).

Plasmid pECS300 was isolated from strain ATCC13032/pECS300 digested with SmaI, religated and used to transform C. glutamicum ATCC13032. Plasmid was isolated from one of the transformants, characterized by restriction mapping and called pECS330.

The E. coli replicon including the β-lactamase resistance gene was deleted from pECS330 by digestion with HindIII, religation and transformation to form the C. glutamicum vector pCV20.

Plasmid pCV20 was digested with SmaI and ligated with the 0.322 Kb PvuII fragment of pUC19 carrying the E. coli lacZ promoter and the multiple cloning site (Yanisch-Perron, C. et al. (1985) Gene 33, 103 pp). C. glutamicum ATCC13032 was transformed with the ligation mixture. Plasmid DNA was isolated from one of the transformants, named pCV22 and its structure confirmed by restriction mapping. The structure of pCV22 including its construction as described above is shown in figure 6.

### 3.4 Cloning of the ppc gene onto the C. glutamicum vector pCV22.

Plasmid pDM2 was isolated from E. coli strain XH11/pDM2 by ethidium bromide CsCl density gradient centrifugation and used to transform C. glutamicum ATCC13032 as described by Yoshihama et al. (J. Bact. 162, 591 pp (1985)). Plasmid DNA was isolated from one of the transformants and shown to have the structure of pDM2.

Plasmid pCV22 was isolated from ATCC13032/pCV22 cut with SalI and treated with calf intestine alkaline phosphatase. Plasmid pDM2 was cut with SalI and SmaI. Both DNA's were mixed, ligated with T4 DNA Ligase and the ligation mixture used to transform C. glutamicum ATCC13032 (Yoshihama, M. et al. (1985) J. Bact. 162, 591 pp). Plasmid DNA designated pDM6 was isolated from one of the transformants and characterized by restriction mapping. The structure of pDM6 is shown in Figure 7.

### 3.5 Measurement of enzyme activity in C. glutamicum clones carrying a recombinant plasmid containing the DNA fragment with the ppc gene.

Phosphoenolpyruvate carboxylase was measured in C. glutamicum strains ATCC13032/pCV22, ATCC13032/pDM2 and ATCC13032/pDM6. The result is shown in Table 3.

| C. glutamicum strains | Phosphoenolpyruvate carboxylase activity (U/mg protein) in the absence of acetyl-CoA |
|---|---|
| ATCC13032/pCV22 | 0.250 |
| ATCC13032/pDM2 | 0.240 |
| ATCC13032/pDM6 | 0.996 |

Table 3: Phosphoenolpyruvate carboxylase activity in dialyzed homogenates obtained from different C. glutamicum strains.

### 4. Effect of plasmid pDM6 on PEP carboxylase activity and on the lysine excretion of the lysine excreting strain C. glutamicum DM58-1.

C. glutamicum strain DM58-1 is a derivative of strain ATCC13032, resistant to 50 mM of the L-lysine analogue S-2-aminoethyl-DL-cysteine, obtained after conventional N-methyl-N'-nitro-N-nitrosoguanidine mutagenesis.

Plasmid pDM6 and as a control plasmid pCV22 were introduced into C. glutamicum DM58-1 to give strains DM58-1/pDM6 and DM58-1/pCV22. Strain DM58-1/pDM6 was deposited at the Deutsche Stammsammlung von Mikroorganismen under DSM4697. The results obtained by measuring the specific PEP carboxylase activity and the concentration of excreted lysine as well as sucrose consumption are shown in Table 4.

The stimulating effect of the elevated level of PEP carboxylase exerted by the cloned ppc gene of plasmid pDM6 on the concentration of lysine excreted and in particular on the yield which is the amount of lysine formed per amount of sucrose consumed is evident.

9

| Strains | PEP carboxylase activity (U/mg protein) | Concentration of L-lysine excreted (g/l) | Yield g L-lysine g sucrose |
|---|---|---|---|
| C. glutamicum DM58-1 | 0.275 | 16..1 | 0,170 |
| C. glutamicum DM58-1/pCV22 | 0.240 | 15.9 | 0,173 |
| C. glutamicum DM58-1/pDM6 | 0.955 | 17.9 | 0,198 |

Table 4: Effect of increased level of PEP carboxylase on lysine excretion by C. glutamicum.

The cultivation was carried out in 100 ml-flasks with indentations containing 10 ml of a medium composed of 12 g/l ammoniumsulfate, 240 g/l molasses, 60 ml/l soy bean protein hydrolysate and 10 g/l $CaCO_3$. In the case of strains DM58-1/pCV22 and DM58-1/pDM6 the medium contained 20 $\mu$g/ml kanamycin. Incubation was carried out for 48h at 30°C and at 300 rpm. After completion of the cultivation lysine in the culture supernatant was quantitatively determined by amino acid analyzers using ion exchange chromatography and ninhydrin detection. Sucrose was quantitatively determined by an enzymatic assay using invertase coupled to hexokinase and glucose-6-phosphat dehydrogenase (Technicon Application Note AAII: Saccharose and Glucose).

**5. Effect of plasmid pDM6 on the threonine and isoleucine excretion of strain B. flavum DM368-2**

B. flavum strain DM368-2 is a derivative of strain ATCC14067, resistant to 4 mg/ml of the threonine analogue $\alpha$-amino-$\beta$-hydroxy-valeric acid, obtained after conventional N-methyl-N'-nitro-N-nitrosoguanidine mutagenesis.

Plasmid pDM6 was isolated from C. glutamicum DM58-1/pDM6 (= DSM4697) and introduced into B. flavum DM368-2 to give strain DM368-2/pDM6. Strain DH368-2 was deposited at the Deutsche Sammlung von Mikroorganismen under DSM 5399. The effect of plasmid pDM6 on the concentration of excreted L-threonine and L-isoleucine as well as sucrose consumption are shown in Table 5.

The stimulating effect of the cloned ppc gene contained in plasmid pDM6 on the concentration of threonine and isoleucine excreted and in particular on the yield which is the amount of amino acid formed per amount of sucrose consumed is evident.

| Strains | Amino acid excreted | Concentration of amino acid excreted g/l | Yield _g amino acid_ g sucrose |
|---|---|---|---|
| B. _flavum_ DM368-2 | L-threonine | 3.32 | 0.0324 |
| | L-isoleucine | 0.92 | 0.0090 |
| B. _flavum_ DM368-2/pDM6 | L-threonine | 3.77 | 0.0370 |
| | L-isoleucine | 1.08 | 0.0106 |

Table 5: Effect of pDM6 on threonine and isoleucine excretion by B. _flavum_.

The cultivation was carried out as described under 4.

Figure 1:   Restriction map of the insert of pTG1200.

Figure 2:   Restriction map of pTG1201.

Figure 3:   Nucleotide sequence of the DNA fragment inserted into pTG1200 containing the ppc gene.

Figure 4:   Restriction map of pZ1.

Figure 5:   Restriction map of pDM2.

Figure 6:   Construction and restriction map of pCV22.

Figure 7:   Restriction map of pDM6.

## Claims

1. Plasmid pDM6, introduced in C.glutamicum DM58-1 which is deposited at the DSM under DSM 4697 characterised by containing a genetic sequence comprising information coding for the production of a protein having the activity of phosphoenolpyruvate carboxylase (PEPC), having a length of 7.9 kb and a restriction map as shown in Fig. 7.

2. A host bacterium belonging to the genus Corynebacterium or Brevibacterium containing the plasmid according to claim 1, producing an L-amino acid selected from the group L-lysine, L-threonine and L-isoleucine.

3. A Corynebacterium according to claim 2 which is selected from the group of those having the identifying characteristics of DSM 4697.

4. A Brevibacterium according to claim 2 which is selected from the group of those having the identifying characteristics of DSM 5399.

5. A method of producing L-amino acid selected from L-Lysine, L-threonine, L-isoleucine by fermentation which comprises culturing in an appropriate medium a bacterium of claims 2, 3 or 4 and recovering the L-amino acid from said medium.

## Patentansprüche

1. Plasmid pDM6, eingeführt in C.glutamicum DM58-1, das unter der Nummer DSM 4697 bei der DSM hinterlegt ist, dadurch gekennzeichnet, daß es eine Informationscodierung für die Herstellung eines Proteins mit der Aktivität von Phosphoenolpyruvat-Carboxylase (PEPC) umfassende genetische Sequenz enthält, wobei das besagte Plasmid, dessen Restriktionskarte in Bild 7 dargestellt ist, eine Länge von 7,9 kb hat.

**EP 0 358 940 B1**

2. Ein Wirtsbacterium des Genus Corynebacterium oder Brevibacterium, das das eine aus der Gruppe L-Lysin, L-Threonin und L-Isoleucin ausgewählte L-Aminosäure erzeugende Plasmid nach Anspruch 1 enthält.

3. Ein Corynebacterium nach Anspruch 2, das aus der Gruppe ausgewählt ist, die die Kennzeichen von DSM 4697 aufweisen.

4. Ein Brevibacterium nach Anspruch 2, das aus der Gruppe ausgewählt ist, die die Kennzeichen von DSM 5399 aufweisen.

5. Ein Verfahren zur Herstellung von aus der Gruppe L-Lysin, L-Threonin und L-Isoleucin ausgewählten L-Aminosäure durch Fermentation, wobei sich das besagte Verfahren auf Kultivierung in einem entsprechenden Medium eines Bacteriums nach den Ansprüchen 2, 3 oder 4 und Gewinnung der L-Aminosäure aus dem besagten Medium erstreckt.

**Revendications**

1. Plasmide pDM6 introduit dans une souche DM58-1 de C. glutamicum qui est déposée à la DSM sous le numéro DSM 4697, caractérisé en ce qu'il contient une séquence génétique comprenant des informations codant pour la production d'une protéine ayant l'activité de la carboxylase de phosphoénolpyruvate (PEPC), ayant une longueur de 7,9 kb et une carte de restriction telle que représentée dans la Fig. 7.

2. Bactérie hôte appartenant au genre Corynebacterium ou Brevibacterium contenant le plasmide selon la revendication 1, produisant un acide L-aminé choisi dans le groupe de la L-lysine, de la L-thréonine et de la L-isoleucine.

3. Souche Corynebacterium selon la revendication 2, sélectionnée dans le groupe de celles ayant les caractéristiques d'identification du DSM 4697.

4. Souche Brevibacterium selon la revendication 2, sélectionnée dans le groupe de celles ayant les caractéristiques d'identification du DSM 5399.

5. Procédé de production d'acide L-aminé choisi parmi la L-lysine, la L-thréonine, la L-isoleucine par fermentation, le procédé consistant à cultiver dans un milieu approprié selon la revendication 2, 3 ou 4 et à récupérer l'acide L-aminé dudit milieu.

**FIGURE 1** : RESTRICTION MAP OF THE INSERT OF PLASMID pTG1200

Legends to symbols: Open bar: 5 kb insert; dashed bars: polylinker
of pUC18 vector. H, HindIII; Sa, SalI; B, BamHI; E, EcoRI;
B/S, BamHI/Sau3A hybrid.

FIGURE 2 : RESTRICTION MAP OF PLASMID pTG1201

Thick line: 3.5 kb SalI fragment bearing the ppc ORF.
Thin line : pBR322 vector. ▶ indicates position and
orientation of the promoter of the tetracycline
resistance determinant.

EP 0 358 940 B1

FIGURE 3 : NUCLEOTIDE SEQUENCE OF THE INSERT OF PLASMID pTG1200

The predicted amino acid sequence corresponding to phosphoenol pyruvate carboxylase is indicated underneath the corresponding DNA sequence.

```
GATCACCAGCAGGCAATCGGCACTGTTCAGAAGCTTGCATTCGCCCTTCCAAAGGAATACTTCGAGAAGGTTGACGTTGCAGTCACCGTTCCTTTCACTG
----.----+----.----+----.----+----.----+----.----+----.----+----.----+----.----+----.----+----.----+  100

ACATCCGCTCCGTCCAGACTCTCGTTGAGGGCGACAAGCTTGAGGTCACTTTCGGTGCTCAGGACGTCTCCCAGCACGAGTCCGGTGCGTACACCGGTGA
----.----+----.----+----.----+----.----+----.----+----.----+----.----+----.----+----.----+----.----+  200

AGTTTCTGCAAGCATGCTGGCAAAGTTGAACTGCTCTTGGGTTGTCGTTGGACACTCCGAGCGCCGCGAGTACCACAACGAGTCTGATGAGTTGGTTGCT
----.----+----.----+----.----+----.----+----.----+----.----+----.----+----.----+----.----+----.----+  300

GCGAAGGCAAAGGCAGCTCTGTCCAACGGCATCAGCCCGATCGTCTGCGTTGGTGAGCCACTGGAAATCCGTGAAGCTGGCACCCACGTTGAGTACGTCG
----.----+----.----+----.----+----.----+----.----+----.----+----.----+----.----+----.----+----.----+  400

TCGAGCAGACCCGTAAGTCCCTTGCTGGCCTGGATGCTGCTGAGCTGGCCAACACCGTTATCGCGTATGAGCCAGTGTGGGCTATCGGCACCGGTAAGGT
----.----+----.----+----.----+----.----+----.----+----.----+----.----+----.----+----.----+----.----+  500

TGCTTCCGCAGCTGACGCTCAGGAAGTGTGCAAGGCTATCCGCGGTCTGATCGTGGAGCTTGCAGGCGACGAGGTCGCTGAGGGCCTGCGTATTCTTTAC
----.----+----.----+----.----+----.----+----.----+----.----+----.----+----.----+----.----+----.----+  600

GGTGGTTCTGTTAAGGCAGAAACCGTCGCTGAGATCGTCGGTCAGCCTGACGTCGACGGCGGACTTGTCGGTGGCGCTTCCCTCGACGGTGAAGCATTCG
----.----+----.----+----.----+----.----+----.----+----.----+----.----+----.----+----.----+----.----+  700
```

Figure 3 continued (b)

```
CCAAGCTGGCTGCCAACGCTGCGAGCGTTGCTTAAAGTACAGAGCTTTAAAGCACAGCCTTAAAGCACAGCCTTAAAGCACAAGCACTGTAGAAGTGCGG
----.----+----.----+----.----+----.----+----.----+----.----+----.----+----.----+----.----+----.----+   800


TTTTGATGAGCCCATGAAAGCCATCGAAATCAATCGCCCAGCTAAACACCTGTTTTGCTGGGTGATTTTTTATCTCATGCACGCCAACACCCTCAATGTG
----.----+----.----+----.----+----.----+----.----+----.----+----.----+----.----+----.----+----.----+   900


AAAGAGTGTTTAAAGTAGTTATGACTGATTTTTTACGCGATGACATCAGGTTCCTCGGTCAAATCCTCGGTGAGGTAATTGCGGAACAAGAAGGCCAGGA
                         m  t  d  f  l  r  d  d  i  r  f  l  g  q  i  l  g  e  v  i  a  e  q  e  g  q  e
----.----+----.----+----.----+----.----+----.----+----.----+----.----+----.----+----.----+----.----+  1000


GGTTTATGAACTGGTCGAACAAGCGCGCCTGACTTCTTTTGATATCGCCAAGGGCAACGCCGAAATGGATAGCCTGGTTCAGGTTTTCGACGGCATTACT
 v  y  e  l  v  e  q  a  r  l  t  s  f  d  i  a  k  g  n  a  e  m  d  s  l  v  q  v  f  d  g  i  t
----.----+----.----+----.----+----.----+----.----+----.----+----.----+----.----+----.----+----.----+  1100


CCAGCCAAGGCAACACCGATTGCTCGCGCATTTTCCCACTTCGCTCTGCTGGCTAACCTGGCGGAAGACCTCTACGATGAAGAGCTTCGTGAACAGGCTC
 p  a  k  a  t  p  i  a  r  a  f  s  h  f  a  l  l  a  n  l  a  e  d  l  y  d  e  e  l  r  e  q  a  l
----.----+----.----+----.----+----.----+----.----+----.----+----.----+----.----+----.----+----.----+  1200


TCGATGCAGGCGACACCCCTCCGGACAGCACTCTTGATGCCACCTGGCTGAAACTCAATGAGGGCAATGTTGGCGCAGAAGCTGTGGCCGATGTGCTGCG
 d  a  g  d  t  p  p  d  s  t  l  d  a  t  w  l  k  l  n  e  g  n  v  g  a  e  a  v  a  d  v  l  r
----.----+----.----+----.----+----.----+----.----+----.----+----.----+----.----+----.----+----.----+  1300


CAATGCTGAGGTGGCGCCGGTTCTGACTGCGCACCCAACTGAGACTCGCCGCCGCACTGTTTTTGATGCGCAAAAGTGGATCACCACCCACATGCGTGAA
 n  a  e  v  a  p  v  l  t  a  h  p  t  e  t  r  r  r  t  v  f  d  a  q  k  w  i  t  t  h  m  r  e
----.----+----.----+----.----+----.----+----.----+----.----+----.----+----.----+----.----+----.----+  1400
```

EP 0 358 940 B1

Figure 3 continued (c)

```
CGCCACGCTTTGCAGTCTGCGGAGCCTACCGCTCGTACGCAAAGCAAGTTGGATGAGATCGAGAAGAACATCCGCCGTCGCATCACCATTTTGTGGCAGA
 r  h  a  l  q  s  a  e  p  t  a  r  t  q  s  k  l  d  e  i  e  k  n  i  r  r  r  i  t  i  l  w  q  t
....·....+....·....+....·....+....·....+....·....+....·....+....·....+....·....+....·....+....·....+  1500

CCGCGTTGATTCGTGTGGCCCGCCCACGTATCGAGGACGAGATCGAAGTAGGGCTGCGCTACTACAAGCTGAGCCTTTTGGAAGAGATTCCACGTATCAA
   a  l  i  r  v  a  r  p  r  i  e  d  e  i  e  v  g  l  r  y  y  k  l  s  l  l  e  e  i  p  r  i  n
....·....+....·....+....·....+....·....+....·....+....·....+....·....+....·....+....·....+....·....+  1600

CCGTGATGTGGCTGTTGAGCTTCGTGAGCGTTTCGGCGAGGGTGTTCCTTTGAAGCCCGTGGTCAAGCCAGGTTCCTGGATTGGTGGAGACCACGACGGT
  r  d  v  a  v  e  l  r  e  r  f  g  e  g  v  p  l  k  p  v  v  k  p  g  s  w  i  g  g  d  h  d  g
....·....+....·....+....·....+....·....+....·....+....·....+....·....+....·....+....·....+....·....+  1700

AACCCTTATGTCACCGCGGAAACAGTTGAGTATTCCACTCACCGCGCTGCGGAAACCGTGCTCAAGTACTATGCACGCCAGCTGCATTCCCTCGAGCATG
 n  p  y  v  t  a  e  t  v  e  y  s  t  h  r  a  a  e  t  v  l  k  y  y  a  r  q  l  h  s  l  e  h  e
....·....+....·....+....·....+....·....+....·....+....·....+....·....+....·....+....·....+....·....+  1800

AGCTCAGCCTGTCGGACCGCATGAATAAGGTCACCCCGCAGCTGCTTGCGCTGGCAGATGCAGGGCACAACGACGTGCCAAGCCGCGTGGATGAGCCTTA
   l  s  l  s  d  r  m  n  k  v  t  p  q  l  l  a  l  a  d  a  g  h  n  d  v  p  s  r  v  d  e  p  y
....·....+....·....+....·....+....·....+....·....+....·....+....·....+....·....+....·....+....·....+  1900

TCGACGCGCCGTCCATGGCGTTCGCGGACGTATCCTCGCGACGACGGCCGAGCTGATCGGCGAGGACGCCGTTGAGGGCGTGTGGTTCAAGGTCTTTACT
 r  r  a  v  h  g  v  r  g  r  i  l  a  t  t  a  e  l  i  g  e  d  a  v  e  g  v  w  f  k  v  f  t
....·....+....·....+....·....+....·....+....·....+....·....+....·....+....·....+....·....+....·....+  2000

CCATACGCATCTCCGGAAGAATTCTTAAACGATGCGTTGACCATTGATCATTCTCTGCGTGAATCCAAGGACGTTCTCATTGCCGATGATCGTTTGTCTG
 p  y  a  s  p  e  e  f  l  n  d  a  l  t  i  d  h  s  l  r  e  s  k  d  v  l  i  a  d  d  r  l  s  v
....·....+....·....+....·....+....·....+....·....+....·....+....·....+....·....+....·....+....·....+  2100
```

EP 0 358 940 B1

Figure 3 continued (d)

```
TGCTGATTTCTGCCATCGAGAGCTTTGGATTCAACCTTTACGCACTGGATCTGCGCCAAAACTCCGAAAGCTACGAGGACGTCCTCACCGAGCTTTTCGA
  l  i  s  a  i  e  s  f  g  f  n  l  y  a  l  d  l  r  q  n  s  e  s  y  e  d  v  l  t  e  l  f  e
  ----.----+----.----+----.----+----.----+----.----+----.----+----.----+----.----+----.----+----.----+   2200

ACGCGCCCAAGTCACCGCAAACTACCGCGAGCTGTCTGAAGCAGAGAAGCTTGAGGTGCTGCTGAAGGAACTGCGCAGCCCTCGTCCGCTGATCCCGCAC
  r  a  q  v  t  a  n  y  r  e  l  s  e  a  e  k  l  e  v  l  l  k  e  l  r  s  p  r  p  l  i  p  h
  ----.----+----.----+----.----+----.----+----.----+----.----+----.----+----.----+----.----+----.----+   2300

GGTTCAGATGAATACAGCGAGGTCACCGACCGCGAGCTCGGCATCTTCCGCACCGCGTCGGAGGCTGTTAAGAAATTCGGGCCACGGATGGTGCCTCACT
  g  s  d  e  y  s  e  v  t  d  r  e  l  g  i  f  r  t  a  s  e  a  v  k  k  f  g  p  r  m  v  p  h  c
  ----.----+----.----+----.----+----.----+----.----+----.----+----.----+----.----+----.----+----.----+   2400

GCATCATCTCCATGGCATCATCGGTCACCGATGTGCTCGAGCCGATGGTGTTGCTCAAGGAATTCGGACTCATCGCAGCCAACGGCGACAACCCACGCGG
  i  i  s  m  a  s  s  v  t  d  v  l  e  p  m  v  l  l  k  e  f  g  l  i  a  a  n  g  d  n  p  r  g
  ----.----+----.----+----.----+----.----+----.----+----.----+----.----+----.----+----.----+----.----+   2500

CACCGTCGATGTCATCCCACTGTTCGAAACCATCGAAGATCTCCAGGCCGGCGCCGGAATCCTCGACGAACTGTGGAAAATTGATCTCTACCGCAACTAC
  t  v  d  v  i  p  l  f  e  t  i  e  d  l  q  a  g  a  g  i  l  d  e  l  w  k  i  d  l  y  r  n  y
  ----.----+----.----+----.----+----.----+----.----+----.----+----.----+----.----+----.----+----.----+   2600

CTCCTGCAGCGCGACAACGTCCAGGAAGTCATGCTCGGTTACTCCGATTCCAACAAGGATGGCGGATATTTCTCCGCAAACTGGGCGCTTTACGACGCGG
  l  l  q  r  d  n  v  q  e  v  m  l  g  y  s  d  s  n  k  d  g  g  y  f  s  a  n  w  a  l  y  d  a  e
  ----.----+----.----+----.----+----.----+----.----+----.----+----.----+----.----+----.----+----.----+   2700
```

EP 0 358 940 B1

Figure 3 continued (e)

```
AACTGCAGCTCGTCGAACTATGCCGATCAGCCGGGGTCAAGCTTCGCCTGTTCCACGGCCGTGGTGGCACCGTCGGCCGCGGTGGCGGACCTTCCTACGA
  l  q  l  v  e  l  c  r  s  a  g  v  k  l  r  l  f  h  g  r  g  g  t  v  g  r  g  g  g  p  s  y  d
----.----+----.----+----.----+----.----+----.----+----.----+----.----+----.----+----.----+----.----+  2800


CGCGATTCTTGCCCAGCCCAGGGGGGGCTGTCCAAGGTTCCGTGCGCATCACCGAGCAGGGCGAGATCATCTCCGCTAAGTACGGCAACCCCGAAACCGCG
  a  i  l  a  q  p  r  g  a  v  q  g  s  v  r  i  t  e  q  g  e  i  i  s  a  k  y  g  n  p  e  t  a
----.----+----.----+----.----+----.----+----.----+----.----+----.----+----.----+----.----+----.----+  2900


CGCCGAAACCTCGAAGCCCTGGTCTCAGCCACGCTTGAGGCATCGCTTCTCGACGTCTCCGAACTCACCGATCACCAACGCGCGTACGACATCATGAGTG
  r  r  n  l  e  a  l  v  s  a  t  l  e  a  s  l  l  d  v  s  e  l  t  d  h  q  r  a  y  d  i  m  s  e
----.----+----.----+----.----+----.----+----.----+----.----+----.----+----.----+----.----+----.----+  3000


AGATCTCTGAGCTCAGCTTGAAGAAGTACGCCTCCTTGGTGCACGAGGATCAAGGCTTCATCGATTACTTCACCCAGTCCACGCCCGCTGCAGGAGATTGG
  i  s  e  l  s  l  k  k  y  a  s  l  v  h  e  d  q  g  f  i  d  y  f  t  q  s  t  p  l  q  e  i  g
----.----+----.----+----.----+----.----+----.----+----.----+----.----+----.----+----.----+----.----+  3100


ATCCCTCAACATCGGATCCAGGCCTTCCTCACGCAAGCAGACCTCCTCGGTGGAAGATTTGCGAGCCATCCCATGGGTGCTCAGCTGGTCACAGTCTCGT
  s  l  n  i  g  s  r  p  s  s  r  k  q  t  s  s  v  e  d  l  r  a  i  p  w  v  l  s  w  s  q  s  r
----.----+----.----+----.----+----.----+----.----+----.----+----.----+----.----+----.----+----.----+  3200


GTCATGCTGCCAGGCTGGTTTGGTGTCGGAACCGCATTAGAGCAGTGGATTGGCGAAGGGGAGCAGGCCACCCAACGCATTGCCGAGCTGCAAACACTCA
  v  m  l  p  g  w  f  g  v  g  t  a  l  e  q  w  i  g  e  g  e  q  a  t  q  r  i  a  e  l  q  t  l  n
----.----+----.----+----.----+----.----+----.----+----.----+----.----+----.----+----.----+----.----+  3300
```

EP 0 358 940 B1

Figure 3 continued (f)

```
ATGAGTCCTGGCCATTTTTCACCTCAGTGTTGGATAACATGGCTCAGGTGATGTCCAAGGCAGAGCTGCGTTTGGCAAAGCTCTACGCAGACCTGATCCC
   e  s  w  p  f  f  t  s  v  l  d  n  m  a  q  v  m  s  k  a  e  l  r  l  a  k  l  y  a  d  l  i  p
----.----+----.----+----.----+----.----+----.----+----.----+----.----+----.----+----.----+----.----+ 3400


AGATACGGAAGTAGCCGAGCGAGTCTATTCCGTCATCCGCGAGGAGTACTTCCTGACCAAGAAGATGTTCTGCGTAATCACCGGCTCTGATGATCTGCTT
   d  t  e  v  a  e  r  v  y  s  v  i  r  e  e  y  f  l  t  k  k  m  f  c  v  i  t  g  s  d  d  l  l
----.----+----.----+----.----+----.----+----.----+----.----+----.----+----.----+----.----+----.----+ 3500


GATGACAACCCACTTCTCGCACGCTCTGTCCAGCGCCGATACCCCTACCTGCTTCCACTCAACGTGATCCAGGTAGAGATGATGCGACGCTACCGAAAAG
 d  d  n  p  l  l  a  r  s  v  q  r  r  y  p  y  l  l  p  l  n  v  i  q  v  e  m  m  r  r  y  r  k  g
----.----+----.----+----.----+----.----+----.----+----.----+----.----+----.----+----.----+----.----+ 3600


GCGACCAAAGCGAGCAAGTGTCCCGCAACATTCAGCTGACCATGAACGGTCTTTCCACTGCGCTGCGCAACTCCGGCTAGTCCAGCCGGCTGGGTAGTAC
  d  q  s  e  q  v  s  r  n  i  q  l  t  m  n  g  l  s  t  a  l  r  n  s  g  .
----.----+----.----+----.----+----.----+----.----+----.----+----.----+----.----+----.----+----.----+ 3700


TCGTGTATACTGTCTAAAGTTATTCGAAATCAGGTGGGCATAAGGTTCACCTGGGTTCTCAAACGGCAAAGGAACATTTTCCACATGGCATTGACGCTTC
----.----+----.----+----.----+----.----+----.----+----.----+----.----+----.----+----.----+----.----+ 3800


AAATCATCCTCGTCGTCGCCAGCCTGCTCATGACGGTTTTCGTCTTGCTGCACAAGGGCAAAGGCGGCGGACTCTCCAGCCTCTTCGGTGGCGGTGTGCA
----.----+----.----+----.----+----.----+----.----+----.----+----.----+----.----+----.----+----.----+ 3900


GTCCAATCTTTCGGGCTCCACTGTTGTTGAAAAGAACCTGGATCGCGTCACCATTTTGGTTGCCGTTATCTGGATTGTGTGCATTGTCGCACTCAACCTC
----.----+----.----+----.----+----.----+----.----+----.----+----.----+----.----+----.----+----.----+ 4000
```

EP 0 358 940 B1

Figure 3 continued (g)

```
ATCCAGACTTATTCATAAGACACGAGCTTAAAAAGAGCGGTTCCCTTTTCATAGGGGAGCCGCTTTTTTGGGTTTTGTCGACCTGTTGTCTCCCCACTGT
----.----+----.----+----.----+----.----+----.----+----.----+----.----+----.----+----.----+----.----+   4100


TCCTCGGTGTGCACTTTCGACACCAAGATTTCGGCAAAGTGGTGGTCAAAATTGGAAAATCTTGGTGCCTAATTCACATACATTCCAATTTTCCCTAAGG
----.----+----.----+----.----+----.----+----.----+----.----+----.----+----.----+----.----+----.----+   4200


ACATCTTTAAAGGGGAACTGTTTCCCGACGGAACGTGGAGTCTATAAAACCGCAGGTTAAAACGCTGCCATAGGGGATTTTTCGGCTGGGGAGACGTGGT
----.----+----.----+----.----+----.----+----.----+----.----+----.----+----.----+----.----+----.----+   4300


GTAAGTGCGGGTTAAAAACGTGACCTTCGTTATAAAAACAGAAATCTATAGAACGATAGGTAAAAACTGGACTAGGTTTATCTATAGCGGAATAGAAAAT
----.----+----.----+----.----+----.----+----.----+----.----+----.----+----.----+----.----+----.----+   4400


ACTCCGCTCGACAGCATCACTTAGCTGAAAGGCCTTTAACATGGACCCCTCAGATCTAGCCTGGATTCTCGCAGCTTTTGCGTTGGTAAGCCTGATGTTC
----.----+----.----+----.----+----.----+----.----+----.----+----.----+----.----+----.----+----.----+   4500


CCCGGATTGTCCCTGCTCTACGGCGGCATGCTGGGTGGGCAACACGTTCTTAACACGTTCATGATGGTTATGAGCTCACTTGGAATCATCAGCCTTGTGT
----.----+----.----+----.----+----.----+----.----+----.----+----.----+----.----+----.----+----.----+   4600


ACATCATTTATGGACACGGACTTGTCTTAGGAAACTCCATCGGTGGGTGGGGAATTATCGGAAATCCCCTTGAATACTTCGGCTTCCGCAACATTATGGA
----.----+----.----+----.----+----.----+----.----+----.----+----.----+----.----+----.----+----.----+   4700


AGATGACGGCACCGGAGACCTCATGTGGGCCGGCTTCTACATTCTGTTCGCTGCAATCTCACTCGCACTTGTTTCATCTGGTGCAGCGGGGCGCATGCGC
----.----+----.----+----.----+----.----+----.----+----.----+----.----+----.----+----.----+----.----+   4800


TTTGGAGCGTGGCTGGTCTTCGGTGTCCTGTGGTTCACCTTTGTGTACGCGCCACTGGCACACTGGGTTTTCGCTATCGATGATC
----.----+----.----+----.----+----.----+----.----+----.----+----.----+----.----+----.   4885
```

EP 0 358 940 B1

Figure 4: Restriction map of pZ1.

Figure 5: Restriction map of pDM2.

23

Figure 6:

Figure 6 contd.:

Figure 6 contd.: Construction and restriction map
                 of pCV22. Legends to symbols:


B       :  BamHI
C       :  BclI
E       :  EcoRI
G       :  BglII
H       :  HindIII
K       :  KpnI
M       :  SmaI
O       :  XhoI
P       :  PstI
S       :  SalI
T       :  Tth111I
V       :  PvuII
X       :  XbaI
II      :  BstEII


Ap      :  Ampicillin resistance gene
Km      :  Kanamycin resistance gene

$P_{lac}$   :  Promoter of E.coli lac operon

kb      :  Kilo base pairs

Figure 7: Restriction map of pDM6. See figure 6
for legends to symbols.